# EUROPÄISCHE PATENTSCHRIFT

(11) **EP 1 216 046 B1**
(45) Veröffentlichungstag und Bekanntmachung des Hinweises auf die Patenterteilung: **04.06.2003**
(21) Anmeldenummer: 00969303.7
(22) Anmeldetag: 26.09.2000
(51) Int. Cl.: A61K 31/56, A61K 31/55, A61K 31/455

(54) **NEUE KOMBINATION VON LOTEPREDNOL UND ANTIHISTAMINIKA**
NOVEL COMBINATION OF LOTEPREDNOL AND ANTIHISTAMINES
NOUVELLE ASSOCIATION DE LOTEPREDNOL ET DE PRODUITS ANTIHISTAMINIQUES

(30) Priorität: 30.09.1999 DE 19947234
(43) Veröffentlichungstag der Anmeldung: 26.06.2002
(73) Patentinhaber: VIATRIS GmbH & Co. KG, 60314 Frankfurt am Main (DE)
(72) Erfinder: SZELENYI, Istvan, 90571 Schwaig (DE); MARX, Degenhard, 01445 Radebeul (DE); HEER, Sabine, 01445 Radebeul (DE); ENGEL, Jürgen, 63755 Alzenau (DE)
(74) Vertreter: Wibbelmann, Jobst, Dr., Dipl.-Chem.
(86) Internationale Anmeldenummer: EP0009391
(87) Internationale Veröffentlichungsnummer: WO01022955

(56) Entgegenhaltungen:
- EP-A- 0 709 099
- WO-A-97/01337

## Beschreibung

Die vorliegende Erfindung betrifft eine neue Kombination von Loteprednol, und mindestens einem Antihistaminikum, wie z.B. Azelastin und/oder Levocabastin, für die simultane, sequentielle oder separate Applikation bei der lokalen Behandlung von Allergien und Atemwegserkrankungen, beispielsweise der allergischen Rhinitis (Rhinokonjunktivitis).

### Hintergrund der Erfindung

Weltweit nimmt die Anzahl der allergischen Erkrankungen stark zu. Studien haben ergeben, daß weltweit durchschnittlich 7,5 % aller Kinder und Jugendlichen an Rhinokonjunktivitis (Heuschnupfen kombiniert mit einer Augensymptomatik) leiden (Worldwide variation in prevalence of symptoms of asthma, allergic rhinoconjunctivitis and atopic eczema: ISAAC, Lancet, 351, 1225-1332, 1998). In westeuropäischen Ländern ist die Prävalenz mit ca. 14 % deulich höher (Annesi-Maesano I. and Oryszczyn MP.: Rhinitis in adolescents, Results of the ISAAC survey, Revue Francaise d'Allergologie et d'lmmunologie Clinique, 38, 283-289, 1998; Norrman E., Nystrom L, Jonsson E and Stjernberg N: Prevalence and incidence of asthma and rhinoconjunctivitis in Swedish teenagers, European Journal of Allergy and Clinical Immunology, 53, 28-35, 1998). Trotz intensiver Forschungsaktivitäten ist die Pathogenese der Rhinokonjunktivitis immer noch nicht vollständig geklärt. Auch wenn in den vergangenen Jahren deutliche Fortschritte in der medikamentösen Behandlung dieser Erkrankung erzielt wurden, ist die Therapie immer noch nicht zufriedenstellend. Die akuten Symptome (Juckreiz, Rötung, Schwellung, Nasen- bzw. Tränenfluß) der Rhinokonjunktivitis können u.a. mit Hilfe von Antihistaminika gut beherrscht werden. Jedoch haben sie kaum einen therapeutisch relevanten Einfluß auf die der Erkrankung zugrunde liegende und stets fortschreitende Entzündung. Oft wird die allergische Rhinitis (Rhinokonjunktivitis) sowohl von Patienten als auch vom Arzt als eine Bagatellerkrankung angesehen und dementsprechend nur unzureichend behandelt. In der Folge kann es jedoch zu einem sog. Etagenwechsel kommen, d.h. aus der relativ harmlosen Rhinitis entwickelt sich eine sehr ernst zu nehmendes Asthma bronchiale. Aus diesem Grunde ist es unerläßlich, bereits die allergische Rhinokonjunktivitis ausreichend und intensiv zu behandeln. Nur dann können die Patienten beschwerdefrei leben und nur dann kann ein u.U. lebensbedrohlicher Etagenwechsel verhindert werden.

Häufig ist es für den behandelnden Arzt in Grenzfällen nicht mit letzter Sicherheit festzustellen, ob noch "nur" eine Rhinokonjunktivitis oder bereits eine Atemwegserkrankung, wie Asthma bronchiale, vorliegt. Vorteilhaft ist, daß die erfindungsgemäße Kombination auch zur Behandlung von Erkrankungen der oberen und unteren Atemwege eingesetzt werden kann.

Zum gegenwärtigen Zeitpunkt können die Corticosteroide die der Rhinokonjunktivitis zugrunde liegende Entzündung am wirksamsten bekämpfen. Viele Patienten aber auch Ärzte setzen jedoch diese Medikamente wegen ihrer möglichen systemischen Nebenwirkungen (z.B. Wachstumsverlangsamung, Osteoporose) überhaupt nicht oder nur sehr zögernd, meistens erst in einer späten Phase der Erkrankung ein.

Loteprednol ist ein Steroid, bei dem im Gegensatz zu anderen Corticosteroiden, die meistens erst in der Leber zu pharmakodynamisch inaktiven Metaboliten abgebaut werden, die metabolische Inaktivierung zum Teil bereits an der Stelle ihrer Verabreichung (intranasal, oculär oder intrapulmonal) erfolgt.

Infolge dieser partiellen lokalen Metabolisierung gelangt keine oder nur sehr wenige pharmakodynamisch aktive Substanz in den systemischen Blutkreislauf, so daß praktisch mit den steroidspezifischen Nebenwirkungen nicht zu rechnen ist. Loteprednol ist für die Therapie der allergischen Konjunktivitis und Uveitis bereits zugelassen.

Aus der WO 97/01337 sind Nasensprays oder -tropfen zur Behandlung von allergischer Rhinitis bekannt, die ein topisches Antihistaminikum wie Levocabastin, Azelastin oder Azatadin und ein topisches Nasensteroid wie z.B. Beclomethason, Flunisolid, Dexamethason oder Budesonid enthalten können. Die EP 0 709 099 A2 beschreibt eine wässrige Nasensuspension zur Behandlung von allergischer Rhinitis die unter anderem ein Steroid wie Loteprednol oder ein Antihistaminikum wie Terfenadin enthalten kann. Eine kombinierte Verwendung der Wirkstoffe wird in diesem Dokument nicht vorgeschlagen. Antihistaminika werden in der akuten Phase der allergischen Rhinokonjunktivitis zur Linderung der oft quälenden Symptome eingesetzt. Besonders vorteilhaft ist die topische Applikation dieser Medikamente, da dadurch hohe lokale Konzentrationen vom Wirkstoff aufgebaut werden können ohne mit nennenswerten Nebenwirkungen rechnen zu müssen. Zum gegenwärtigen Zeitpunkt befinden sich zwei lokal verabreichbare Antihistaminika, Azelastin und Levocabastin auf dem Markt. Beide sind hochwirksam und sehr gut verträglich.

Überraschenderweise wurde nun gefunden, daß die neue Kombination von Loteprednol und mindestens einem Antihistaminikum bei der Behandlung von Allergien und/oder Atemwegserkrankungen durch topische Verabreichung vorteilhaft ist. Die Verabreichung kann dabei simultan, sequentiell oder separat erfolgen. Die Erfindung dient der Verbesserung der Therapie von allergischer Rhinitis (Rhinokonjunktivitis). Das Antihistaminikum sorgt für die schnelle Beseitigung der akuten Symptome (z.B. Rötung, Juckreiz, Schwellung). Mit dem in der Kombination enthaltenen Corticosteroid kann die dem Krankheitsbild zugrunde liegenden Entzündung erfolgreich bekämpft werden.

Die Erfindung betrifft somit ein pharmazeutisches Gemisch, umfassend Loteprednol oder einen pharmazeutisch verträglichen Ester davon und mindestens ein Antihistaminikum. Vorzugsweise handelt es sich bei dem Antihistaminikum um ein topisch verabreichbares Antihistaminikum beispielsweise Azelastin und/oder Levocabastin. Der pharmazeutisch verträgliche Ester des Loteprednols ist das Loteprednol Etabonat.

Die Herstellung von Loteprednol und Loteprednol Etabonat ist beispielsweise in dem deutschen Patent Nr. DE 31 26 732, dem korrespondierenden U.S.-Patent Nr. 4,996,335 und dem korrespondierenden japanischen Patent Nr. JP-89 011 037 beschrieben.

Weiterhin betrifft die Erfindung ein Arzneimittel zur Behandlung von Erkrankungen der unteren und/oder oberen Atemwege und/oder zur Behandlung von Allergien, enthaltend als Wirkstoffe Loteprednol und mindestens ein topisch verabreichbares Antihistaminikum, gegebenenfalls zusammen mit üblichen Hilfs- oder Trägerstoffen, zur simultanen, sequenziellen oder separaten Verabreichung.

Das Arzneimittel kann gleichzeitig, nacheinander oder unabhängig von einander intranasal oder intraoculär verabreicht werden. Es handelt sich um eine inhalierbare flüssige oder feste Zubereitung.

Gegenstand der Erfindung ist auch ein Verfahren zur Herstellung eines Arzneimittels zur Behandlung und Prophylaxe von Atemwegserkrankungen und/oder Allergien wie im Anspruch 9 angegeben.

Schließlich betrifft die Erfindung die Verwendung einer Kombination von Loteprednol oder einen pharmazeutisch verträglichen Ester davon und einem Antihistaminikum zur simultanen, sequenziellen oder separaten Verabreichung zur Herstellung eines Arzneimittels zur Behandlung und Prophylaxe von Atemwegserkrankungen und/oder Allergien. Insbesondere betrifft die Erfindung eine Kombination, wie zuvor genannt, zur simultanen, sequenziellen oder separaten Verabreichung zur Herstellung eines Arzneimittels zur Behandlung von allergischer Rhinitis oder Rhinokonjunktivitis.

Azelastin und Levocabastin können auch in Form der pharmazeutisch verträglichen Salze verwendet werden. Bevorzugt sind beispielsweise die Hydrochloride.

Durch die topische Verabreichung der Komponenten (Steroid und Antihistaminikum) können therapeutisch wirksame Konzentrationen bereits bei niedrigen Dosierungen erreicht werden. Die kombinative Gabe beider Substanzen (Antihistaminikum + Loteprednol) ermöglicht die Bekämpfung der lästigen Frühphasenreaktionen wie Juckreiz, Nasenfluß durch das Antihistaminikum und das Fortschreiten der Entzündung durch das Loteprednol. Außerdem wird dadurch die Gefahr des Auftretens von unerwünschten Wirkungen auf ein Minimum reduziert und somit ist eine bessere Compliance der Patienten zu erwarten.

Die vorliegende Erfindung beschreibt eine neue Kombination, in der Loteprednol und ein Antihistaminikum (vorzugsweise Azelastin und/oder Levocabastin) gleichzeitig, hintereinander als Einzelsubstanzen oder als fixe Kombination topisch (intranasal oder intraoculär) gegeben werden. Durch diese Kombination kommt es nicht nur zu einem schnellen Wirkungseintritt, sondern auch zu einer hohen therapeutischen Wirksamkeit, die mit einer starken antientzündlichen Wirkung einhergeht. In einer vorteilhaften Ausführungsform liegendie wirksamen Komponente dieser Kombination in Form einer fixen Kombination vor, wodurch die Anwendung für den Patienten einfacher ist, denn beide Wirkstoffe sind in ein und demselben Behälter enthalten.

Gemäß einer weiteren Ausführungsform der Erfindung kann das Antihistaminikum auch oral verabreicht werden.

Die vorgesehene Dosierung erfolgt zweimal täglich, wobei die Einzeldosis vom Loteprednol zwischen 10 und 500 µg, bevorzugt 50 und 200 µg, liegt. Die Dosis vom Antihistaminikum beträgt 50 - 500 µg, bevorzugt 100 - 200 µg. Die tatsächliche Dosis hängt vom allgemeinen Zustand der Patienten (Alter, Gewicht, etc.) und Schweregrad der Erkrankung ab.

Folgende pharmakologische Untersuchung wurde durchgeführt um die beschriebene Erfindung zu untermauern.

*In vitro* wurden Untersuchungen zur Beeinflussung der Freisetzung des proinflammatorischen Cytokins TNFα im 1:5 verdünnten Humanblut verschiedener Spender durchgeführt. Die Stimulation erfolgte mit Lipopolysaccharid (LPS) von Salmonella abortus equi (10 µg/ml) über 24 h bei 37°C und 5% CO₂ im Brutschrank. Die Bestimmung der TNFα-Freisetzung erfolgte mit einem ELISA, aufgebaut aus Antikörpern der Fa. Pharmingen. Die Ergebnisse wurden als prozentuale Hemmung der LPS-induzierten TNFα-Freisetzung angegeben und sind in der Tabelle 1 dargestellt.

**Tabelle 1**

| Wirkstoff | Konzentration [µmol/l] | Hemmung der TNFα-Freisetzung |
|---|---|---|
| Azelastin | 10 | 2% |
| Loteprednol | 0,001 | 1 % |
| | 0,01 | 2 % |
| | 0,03 | 8 % |
| Azelastin + Loteprednol | 10 + 0,001 | 12 %* |
| | 10 + 0,01 | 18 %* |
| | 10 + 0,03 | 22 %* |

| | | |
|---|---|---|
| * signifikant (p<0.05) | | |

Werden das Antihistaminikum Azelastin oder das "soft" Steroid Loteprednol allein appliziert, bleibt die LPS-induzierte TNFα-Freisetzung praktisch unverändert. In der Anwesenheit von Azelastin (10 µmol/l) wird die TNFα-Freisetzung durch Loteprednol konzentrationsabhängig verstärkt gehemmt.

*In vivo* Untersuchungen wurden an jungen, mit einem Antigen (Extrakt aus Ascaris suum) aktiv sensibilisierten Hausschweinen durchgeführt. Drei Wochen später wurden sie einer Allergen-Provokation ausgesetzt, die durch intranasale Instillation des Ascaris-Extraktes erfolgte. Diese lokale intranasale Allergen-Provoaktion führt zu einem sehr starken Anstieg der nasalen Sekretion (Rhinorrhoe). Die Sekretmenge wurde gravimetrisch erfaßt. Die Ergebnisse sind in der Tabelle 2 zusammengestellt.

**Tabelle 2**

| Wirkstoff | Dosis in µg/Nasenloch | Hemmung der nasalen Sekretion | Anzahl der Tiere |
|---|---|---|---|
| Azelastin | 10 | 15 % | 5 |
| Loteprednol | 20 | 8 % | 5 |
| Azelastin + Loteprednol | 10 + 20 | 48 %* | 5 |

| | | | |
|---|---|---|---|
| * signifikant (p<0.05) | | | |

Wenn das Antihistaminikum Azelastin oder das "soft" Steroid Loteprednol bei den Dosierungen 10 bzw. 20 µg/Nasenloch verwendet wird, kommt es nur zu marginalen Hemmungen der allergisch induzierten nasalen Hypersekretion. Wenn beide Wirkstoffe aber gleichzeitig gegeben werden, wird die Rhinorrhoe um 48 % (signifikant) reduziert.

Für die topische Anwendung können verschiedene pharmazeutische Formulierungen, z.B. Nasensprays, Nasentropfen und Augentropfen, in Frage kommen.

Die vorliegende Erfindung beschreibt eine Kombination, in der ein Steroid, hier Loteprednol, und ein Antihistaminikum, z.B. Azelastin und/oder Levocabastin, gleichzeitig, als Einzelsubstanzen hintereinander oder als fixe Kombination verabreicht werden.

Aufgrund der Wasserlöslichkeit des Wirkstoffes Azelastinhydrochlorid können Formulierungen mit diesem Wirkstoff vorzugsweise als Lösungen formuliert werden. Loteprednoletabonat ist dagegen praktisch wasserunlöslich und wird daher als wässrige Suspension formuliert. In einer Formulierung in der beide Wirkstoffe kombiniert werden, liegt demnach Azelastinhydrochlorid in Wasser gelöst und Loteprednoletabonat in Wasser suspendiert vor.

Neben den wirksamen Bestandteilen Antihistaminikum, z.B. Azelastinhydrochlorid, und Steroid, z.B. Loteprednoletabonat, können die erfindungsgemäßen pharmazeutischen Zubereitungen weitere Bestandteile wie Konservierungsstoffe, Stabilisatoren, Isotonisierungsmittel, Verdickungsmittel, Suspensionsstabilisatoren, Hilfstoffe zur pH-Wert-Einstellung, Puffersysteme und Netzmittel enthalten.

Zum Beispiel kommen als Konservierungsmittel in Frage: Benzalkoniumchlorid, Chlorbutanol, Thiomersal, Methylparaben, Propylparaben, Sorbinsäure und deren Salze, Natriumedetat, Phenylethylalkohol, Chlohexidinhydrochlorid - acetat, - digluconat, Cetylpyridiniumchlorid,-bromid, Chlokresol, Phenylquecksilberacetat, Phenylquecksilbernitrat, Phenyquecksilberborat, Phenoxyethanol.

Für die Konservierung wird vorzugsweise eine Kombination aus Natriumedetat und Benzalkoniumchlorid verwendet. Natriumedetat wird dabei in Konzentrationen von 0,05 - 0,1% und Benzalkoniumchlorid in Konzentrationen von 0,005 - 0,05 % eingesetzt. Auch eine Kombination aus Natriumedetat, Benzalkoniumchlorid und Phenylethylalkohol wird bevorzugt eingesetzt.

Geeignete Hilfsstoffe zur Einstellung der Isotonie der Formulierungen sind beispielsweise: Natriumchlorid, Kaliumchlorid, Mannitol, Glucose, Sorbitol, Glycerol, Propylenglycol. Im Allgemeinen werden diese Hilfsstoffe in Konzentrationen von 0,1 bis 10% eingesetzt.

Die Formulierungen der Erfindung können ebenfalls geeignete Puffersysteme oder andere Hilfsstoffe zur pH-Einstellung beeinhalten um einen pH-Wert einzustellen und aufrechtzuerhalten in der Größenordnung von 4 -8, vorzugsweise von 5 bis 7,5. Geeignete Puffersysteme sind Citrat, Phosphat, Tromethamol, Glycin, Borat, Acetat. Diese Puffersysteme können hergestellt werden aus Substanzen wie, Citronensäure Mononatriumphosphat, Dinatriumphosphat Glycin, Borsäure, Natriumtertaborat, Essigsäure, Natriumactat.
Es können ebenfalls weitere Hilfsstoffe zur pH-Einstellung verwendet werden wie Salzsäure oder Natriumhydroxid.

Um eine stabile wässrige Suspension mit dem wasserunlöslichen Wirkstoff Loteprednoletabonat herzustellen sind weiterhin geeignete Suspensionsstabilisatoren sowie geeignete Netzmittel erforderlich, um den suspendierten Wirkstoff in geeigneter Weise zu dispergieren und zu stabilisieren.

Als Suspensionstabilisatoren kommen wasserlösliche oder teilweise wasserlösliche Polymere in Frage: dazu gehören beispielsweise Methylcellulose (MC), Natriumcarboxymethylcellulose (Na-CMC), Hydroxypropylmethylcellulose (HPMC) Polyvinylalkohol (PVAL), Polyvinypyrrolidon (PVP), Polyacrylsäure, Polyacrylamid, Gellan Gum (Gelrite®) Aluminiumoxydhydrat (Unemul®) Dextrine, Cyclodextrine sowie Mischungen aus Mikrokristalliner Cellulose und Natriumcarboxymethylcellulose (Avicel RC 501®, Avicel RC 581®, Avicel RC 591®, Avicel CL 611®).
Diese Substanzen können gleichzeitig als Verdickungsmittel dienen um die Viskosität zu erhöhen und dadurch den Kontakt der Wirkstoffe mit dem Gewebe am Applikationsort zu verlängern.

Als Netzmittel für die Formulierungen kommen in Frage: Benzalkoniumchlorid, Cetylpyridiniumchlorid, Tyloxapol, verschiedene Polysorbate (Tween ®) sowie weitere polyoxyethylierte Substanzen und Poloxamere.

### Beispiele:

### Beispiel 1:

### Nasenspray mit Azelastinhydrochlorid (0,1%)

| | |
|---|---|
| Azelastinhydrochlorid | 0,1000g |
| Hydroxypropylmethylcellulose | 0,1000g |
| Natriumedetat | 0,0500 g |
| Benzalkoniumchlorid | 0,0125 g |
| Natriumhydoxid | q.s. ph 6,0 |
| Sorbitol Lösung 70% | 6,6666 g |
| Gereinigtes Wasser | ad 100 ml |

### Herstellung der Lösung:

In einem geigneten Rührwerksbehälter ca 45 kg gereinigtes Wasser vorlegen. Darin den Wirkstoff, Hydroxypropylmethylcellulose, Natriumedetat, Benzalkoniumchlorid und Sorbitollösung nacheinander zugeben und unter Rühren auflösen. Die entstandene Lösung mit gereinigtem Wasser auf ein Volumen von 49,5 Liter auffüllen. Den pH-Wert der Lösung mit 1N Natronlauge auf pH 6,0 einstellen. Mit gereinigtem Wasser auf das Endvolumen von 50,0 Liter auffüllen und Rühren. Die Lösung durch ein geignetes Filter filtrieren und in Flaschen abfüllen, welche anschließend mit einer geeigneten Nasenspraypumpe versehen werden.

### Beispiel 2

### Nasenspray-Suspension mit Loteprednoletabonat (1%)

| | |
|---|---|
| Loteprednoletabonat | 1,0000 g |
| Avicel RC 591 | 1,1000 g |
| Polysorbat 80 | 0,1000 g |
| Sorbitol-lösung 70% | 6,0000 g |
| Natriumedetat | 0,0500 g |
| Benzalkoniumchlorid | 0,0200 g |
| Gereinigtes Wasser | ad 100 ml |

### Herstellung:

In einem geeigneten Rührwerksbehälter mit Homogenisiereinrichtung 45 kg gereinigtes Wasser vorlegen und darin Avicel RC 591 hochtourig einhomogenisieren. Danach nacheinander die Stoffe Polysorbat 80, Sorbitol-Lösung, Natriumedetat und Benzalkoniumchlorid unter Rühren auflösen. Anschließend den Wirkstoff Loteprednoletabonat hochtourig einhomogenisieren, bis eine gleichmäßige Suspension entstanden ist. Danach auf das Endvolumen von 50 Liter mit gereinigtem Wasser auffüllen und weiter homogenisieren. Anschließend die Suspension evakuieren um die entstandenen Luftblasen zu entfernen. Die entstandene Suspension wird anschließend in Flaschen abgefüllt, welche danach mit einer geeigneten Nasenspraypumpe versehen werden

### Beispiel 3:

### Nasenspray mit Loteprednoletabonat (1%, suspendiert) und Azelastinhydrochlorid (0,1%, gelöst)

| | |
|---|---|
| Loteprednoletabonat | 1,0000 g |
| Azelastinhydrochlorid | 0,1000 g |
| Avicel RC 591 | 1,1000 g |
| Polysorbat 80 | 0,1000 g |
| Sorbitol-lösung 70% | 6,0000 g |
| Natriumedetat | 0,0500 g |
| Benzalkoniumchlorid | 0,0200 g |
| Gereinigtes Wasser | ad 100 ml |

### Herstellung:

In einem geeigneten Rührwerksbehälter mit Homogenisiereinrichtung 45 kg gereinigtes Wasser vorlegen und darin Avicel RC 591 hochtourig einhomogenisieren. Danach nacheinander den Wirkstoff Azelastinhydrochlorid sowie die Hilfsstoffe Polysorbat 80, Sorbitol-Lösung, Natriumedetat und Benzalkoniumchlorid unter Rühren auflösen.
Anschließend den Wirkstoff Loteprednoletabonat hochtourig einhomogenisieren, bis eine gleichmäßige Suspension entstanden ist. Danach auf das Endvolumen von 50 Liter mit gereinigtem Wasser auffüllen und weiter homogenisieren. Anschließend die Suspension evakuieren um die entstandenen Luftblasen zu entfernen.
Die entstandene Suspension wird anschließend in Flaschen abgefüllt, welche danach mit einer geeigneten Nasenspraypumpe versehen werden.

## Patentansprüche

1. Pharmazeutisches Gemisch, umfassend Loteprednol oder einen pharmazeutisch verträglichen Ester davon und mindestens ein Antihistaminikum.

2. Gemisch nach Anspruch 1, **dadurch gekennzeichnet, daß** es sich bei dem Antihistaminikum um ein topisch verabreichbares Antihistaminikum handelt.

3. Gemisch nach den Ansprüchen 1 oder 2, **dadurch gekennzeichnet, daß** es sich bei dem Antihistaminikum um Azelastin und/oder Levocabastin handelt.

4. Gemisch nach einem der vorhergehenden Ansprüche, **dadurch gekennzeichnet, daß** es sich bei dem pharmazeutisch verträglichen Ester um Loteprednol Etabonat handelt.

5. Arzneimittel zur Behandlung von Erkrankungen der unteren und/oder oberen Atemwege und/oder zur Behandlung von Allergien, enthaltend als Wirkstoffe Loteprednol und mindestens ein topisch verabreichbares Antihistaminikum, gegebenenfalls zusammen mit üblichen Hilfs- oder Trägerstoffen, zur simultanen, sequenziellen oder separaten Verabreichung.

6. Arzneimittel nach Anspruch 5, **dadurch gekennzeichnet, daß** es gleichzeitig, nacheinander oder unabhängig voneinander intranasal oder intraoculär verabreicht werden kann.

7. Arzneimittel nach einem der Ansprüche 5 oder 6, **dadurch gekennzeichnet, daß** es sich um eine inhalierbare flüssige oder feste Zubereitung handelt.

8. Arzneimittel nach Anspruch 5, **dadurch gekennzeichnet, daß** das Antihistaminikum auch oral verabreicht werden kann.

9. Verfahren zur Herstellung eines Arzneimittels zur Behandlung und Prophylaxe von Atemwegserkrankungen und/oder Allergien, enthaltend als Wirkstoffe Loteprednol oder einen pharmazeutisch verträglichen Ester davon und mindestens ein Antihistaminikum, **dadurch gekennzeichnet, daß** man das Loteprednol oder dessen pharmazeutisch verträglichen Ester und den oder die Antihistaminika einzeln oder zusammen, gegebenenfalls zusammen mit üblichen Hilfs- oder Trägerstoffen, vermischt und die so erhaltene Mischung in geeignete Darreichungsformen überführt.

10. Verwendung einer Kombination von Loteprednol oder einem pharmazeutisch verträglichen Ester davon und einem Antihistaminikum zur simultanen, sequenziellen oder separaten Verabreichung zur Herstellung eines Arzneimittels zur Behandlung und Prophylaxe von Atemwegserkrankungen und/oder Allergien.

11. Verwendung nach Anspruch 10 einer Kombination von Loteprednol oder einem pharmazeutisch verträglichen Ester davon und einem Antihistaminikum zur simultanen, sequenziellen oder separaten Verabreichung zur Herstellung eines Arzneimittels zur Behandlung von allergischer Rhinitis und Rhinokonjunktivitis.

## Claims

1. Pharmaceutical mixture comprising loteprednol or a pharmaceutically tolerable ester thereof and at least one antihistamine.

2. Mixture according to claim 1, **characterized in that** the antihistamine is a topically administrable antihistamine.

3. Mixture according to claims 1 or 2, **characterized in that** the antihistamine is azelastine and/or levocabastine.

4. Mixture according to one of the above claims, **characterized in that** the pharmaceutically tolerable ester is loteprednol etabonate.

5. Medicament for the treatment of disorders of the lower and/or upper airways and/or for the treatment of allergies, comprising as active compounds loteprednol and at least one topically administrable antihistamine, if appropriate together with customary excipients or vehicles, for simultaneous, sequential or separate administration.

6. Medicament according to claim 5, **characterized in that** it can be administered intranasally or intraocularly simultaneously, in succession or independently of one another.

7. Medicament according to claims 5 or 6, **characterized in that** it is an inhalable liquid or solid preparation.

8. Medicament according to claim 5, **characterized in that** the antihistamine can also be administered orally.

9. Process for the production of a medicament for the treatment and prophylaxis of airway disorders and/or allergies, comprising as active compounds loteprednol or a pharmaceutically tolerable ester thereof and at least one antihistamine, **characterized in that** the loteprednol or a pharmaceutically tolerable ester thereof and the antihistamine(s) are mixed individually or together, optionally together with customary excipients or vehicles, and the mixture thus obtained is converted into suitable administration forms.

10. Use of a combination of loteprednol or a pharmaceutically tolerable ester thereof and an antihistamine for simultaneous, sequential or separate administration for the production of a medicament for the treatment and prophylaxis of airway disorders and/or allergies.

11. Use according to claim 10 of a combination of loteprednol or a pharmaceutically tolerable ester thereof and an antihistamine for simultaneous, sequential or separate administration for the production of a medicament for the treatment of allergic rhinitis and rhinoconjunctivitis.

## Revendications

1. Mélange pharmaceutique comprenant du loteprednol ou un ester pharmaceutiquement acceptable de celui-ci et au moins un anti-histaminique.

2. Mélange selon la revendication 1 **caractérisé en ce que** l'anti-histaminique est un anti-histaminique pouvant être administré par voie topique.

3. Mélange selon la revendication 1 ou 2 **caractérisé en ce que** l'anti-histaminique est l'azélastine et/ou la lévocabastine.

4. Mélange selon l'une des revendications précédentes **caractérisé en ce que** l'ester pharmaceutiquement acceptable est l'étabonate de loteprednol.

5. Médicament pour le traitement des maladies des voies respiratoires inférieures et/ou supérieures et/ou pour le traitement des allergies, contenant comme principes actifs du loteprednol et au moins un anti-histaminique pouvant être administré par voie topique, éventuellement avec des adjuvants ou supports habituels, pour l'administration simultanée, successive ou séparée.

6. Médicament selon la revendication 5 **caractérisé en ce qu'**il peut être administré par voie intranasale ou intraoculaire simultanément, successivement ou indépendamment.

7. Médicament selon l'une des revendications 5 et 6 **caractérisé en ce qu'**il s'agit d'une préparation liquide ou solide inhalable.

8. Médicament selon la revendication 5 **caractérisé en ce que** l'anti-histaminique peut aussi être administré par voie orale.

9. Procédé de production d'un médicament pour le traitement et la prophylaxie des maladies des voies respiratoires et/ou des allergies, contenant comme principes actifs du loteprednol ou un ester pharmaceutiquement acceptable de celui-ci et au moins un anti-histaminique, **caractérisé en ce que** l'on mélange le loteprednol ou son ester pharmaceutiquement acceptable et le ou les anti-histaminiques isolément ou conjointement, éventuellement avec des adjuvants ou supports habituels, et on convertit le mélange ainsi obtenu en des formes d'administration appropriées.

10. Utilisation d'une combinaison de loteprednol ou d'un ester pharmaceutiquement acceptable de celui-ci et d'un anti-histaminique pour l'administration simultanée, successive ou séparée pour la production d'un médicament pour le traitement et la prophylaxie des maladies des voies respiratoires et/ou des allergies.

11. Utilisation selon la revendication 10 d'une combinaison de loteprednol ou d'un ester pharmaceutiquement acceptable de celui-ci et d'un anti-histaminique pour l'administration simultanée, successive ou séparée pour la production d'un médicament pour le traitement de la rhinite allergique et de la rhinoconjonctivite.
